# EUROPEAN PATENT SPECIFICATION

(11) **EP 1 178 793 B1**
(45) Date of publication and mention of the grant of the patent: **30.11.2005**
(21) Application number: 00920968.5
(22) Date of filing: 03.05.2000
(51) Int. Cl.: A61K 31/401, A61K 9/20

(54) **STABLE SOLID PHARMACEUTICAL COMPOSITIONS CONTAINING ENALAPRIL MALEATE**
ENALAPRILMALEAT ENTHALTENDE, STABILE, FESTE ARZNEIMITTEL
COMPOSITIONS PHARMACEUTIQUE SOLIDE STABLE CONTENANT UN MALEATE D'ENALAPRIL

(30) Priority: 03.05.1999 US 304413
(43) Date of publication of application: 13.02.2002
(73) Proprietor: RANBAXY LABORATORIES, LTD., New Delhi 110 019 (IN)
(72) Inventor: BHUSHAN, Indu, New Delhi 110 019 (IN); SOMANI, Jitendra K., Calcutta 700 045 (IN)
(74) Representative: Gilholm, Stephen Philip
(86) International application number: PCT/IB2000/000569
(87) International publication number: WO 2000/066116

(56) References cited:
- EP-A- 0 795 324
- WO-A-98/26765
- US-A- 4 743 450
- US-A- 5 562 921

## Description

### FIELD OF THE INVENTION

The present invention relates to a stable solid pharmaceutical composition in the form of tablets or capsules comprising enalapril maleate as active ingredient.

### BACKGROUND OF THE INVENTION

The quality of a pharmaceutical dosage form is defined and specified in terms of measurable as well as non-measurable pharmaceutical attributes such as content of active ingredient, percent of impurities, rate at which the active ingredient dissolves, appearance, etc. A stable dosage form is one which retains all its desirable physical and chemical attributes up to a time when the dosage form is consumed by the patient. When a dosage form meets the specifications, of purity and strength of active ingredient up to a time when the dosage form is consumed by the patient it is considered as chemically stable. Strength herein refers to the amount of active ingredient in the dosage form. Chemical instability results in a decrease in the percent of active ingredient and consequently the desired therapeutic efficacy may be compromised.

Moreover, degradation of the active ingredient may result in the formation of degradation products that are undesirable, for example, acetic acid formed as a result of degradation of aspirin has an unacceptable odor; or in the formation of degradation products that could have undesirable toxicity. It is thus quite apparent that stability is an essential component in the design of pharmaceutical dosage forms and that all pharmaceutical dosage forms must be designed to be stable.

Enalapril maleate, an angiotensin converting enzyme inhibitor, known through U.S. Pat. No. 4,374,829, is useful in the treatment of essential and renovascular hypertension. It is highly susceptible to decomposition and undergoes autocyclization to form diketopiperazine. In addition enalapril maleate may form diacids via hydrolysis or may undergo oxidation resulting in discoloration when formulated into pharmaceutical dosage forms.

U.S. Pat. No. 5,562,921 discloses that enalapril maleate degrades at a faster rate in the presence of some diluents namely microcrystalline cellulose, dibasic calcium phosphate, and tribasic calcium phosphate; lubricants, namely magnesium stearate and calcium stearate, and disintegrants such as crosspovidone, and sodium starch glycolate. The disclosed composition was free or substantially free of microcrystalline cellulose, cellulose derivatives or cellulose polymers, calcium phosphate, disintegrants, and magnesium stearate. At least 50% by weight of the pharmaceutical excipients in the composition were pharmaceutically acceptable water soluble substances such that the composition could dissolve sufficiently rapidly and not require the use of disintegrants.

U.S. Pat. No. 4,743,450 assigned to Warner-Lambert Company discloses a stable pharmaceutical composition containing an Angiotensin Converting Enzyme (ACE) inhibitor susceptible to cyclization, hydrolysis and discoloration. The disclosed composition is said to contain an alkali or alkaline earth metal carbonate to minimize cyclization and discoloration and also suitable amount of a saccharide to inhibit hydrolysis of the active ingredient.

U.S. Pat. No. 4,830,853 assigned to Warner-Lambert Company discloses a pharmaceutical composition containing an ACE inhibitor stabilized for oxidation and discoloration using 0.5 -15% w/w of ascorbic acid and/or sodium ascorbate by weight of the combination with the drug and at least one lubricant and/or excipient or mixtures thereof which do not interfere with the functions of stabilizers.

U.S. Pat. No. 4,793,998 assigned to Warner-Lambert Company discloses a pharmaceutical composition containing from about 1 to 70% by weight of an ACE inhibitor and about 1-90% by weight of the stabilizer which contains either ascorbic acid alone or at least 10% w/w of ascorbic acid in combination with organic acids such as fumaric, maleic and citric acid as a cyclization and/or hydrolysis inhibitors with at least one lubricant and/or excipient.

U.S. Pat. No. 5,690,962 assigned to Apotex Corporation discloses a stable formulation made by reacting enalapril maleate with a sodium compound. The composition obtained is said to contain the reaction products enalapril sodium and disodium maleate and was more stable than a similar composition containing enalapril maleate. However, a disadvantage of such processing is that the active ingredient does not retain its chemical identity as it is converted from enalapril maleate to enalapril sodium.

WO 9 826 765 describes pharmaceutical compositions comprising enalapril maleate stabilized by maleic acid. However, there is no description or suggestion in this patent application of using an edible dessicant as a stabilizing agent.

European Patent Application No. 0 795 324 describes a pharmaceutical composition comprising a maximum of 1% by weight of colloidal silica. However, it is known that at low concentrations, colloidal silica acts as a glidant rather than having any effect on degradation by moisture.

In light of the foregoing, a principal object of the present invention is to provide a stable oral pharmaceutical composition in the form of tablets or capsules comprising:
(a) enalapril maleate as the active ingredient;
(b) a stabilizer consisting essentially of an edible dessicant in an amount ranging from 2 to 15% by weight of the total weight of the composition; and
(c) microcrystalline cellulose as an excipient.

Use of maleic acid or an edible desiccant as one of the pharmaceutical excipients significantly reduces the rate of degradation of enalapril maleate in the formulation.

The pharmaceutical composition according to the present invention provides excellent stability in spite of the presence of several destabilizing excipients and thus provides greater flexibility in the choice of excipients for the formulation of enalapril maleate tablets.

According to the present invention, the pharmaceutical composition contains maleic acid and an edible dessicant or only an edible dessicant. Enalapril maleate is an ester of enalapril and maleic acid. Maleic acid, a dicarboxylic acid, is commonly used in food products and beverages. It is acceptable for use as a pharmaceutical excipient/aid and is useful as an acidifying agent, a lubricant, and taste enhancer. Maleic acid may be present in an amount from 0.1% to 5% preferably from 0.1% to 1 % by weight of the total weight of the composition.

According to another aspect of the present invention, the pharmaceutical composition in the form of tablets contains an edible desiccant within the tablet itself. Suitable desiccants that can be used in the present invention are those that are edible, and include, for example, edible grades of silica gel, crystalline sodium, potassium or calcium aluminosilicate, colloidal silica, anhydrous calcium sulphate and the like. The desiccant may be present in an amount from about 1% to 20%, preferably from about 2% to 15% by weight of the total weight of the composition.

According to the present invention, the pharmaceutical composition also contains conventional pharmaceutical excipients that are well known to those skilled in the art including diluents, binders, disintegrants, lubricants, coloring agents, and others. Pharmaceutical excipients that may be used in the present invention may include diluents such as microcrystalline cellulose, lubricants such as magnesium stearate, and disintegrants such as cross-linked carboxy methyl cellulose sodium, which have been reported to be incompatible with enalapril maleate in prior art references.

### DETAILED DESCRIPTION OF THE INVENTION

### EXAMPLE 1

This example illustrates the process for the preparation of a composition containing an edible desiccant. Tablets were prepared according to the composition given in Table 1.

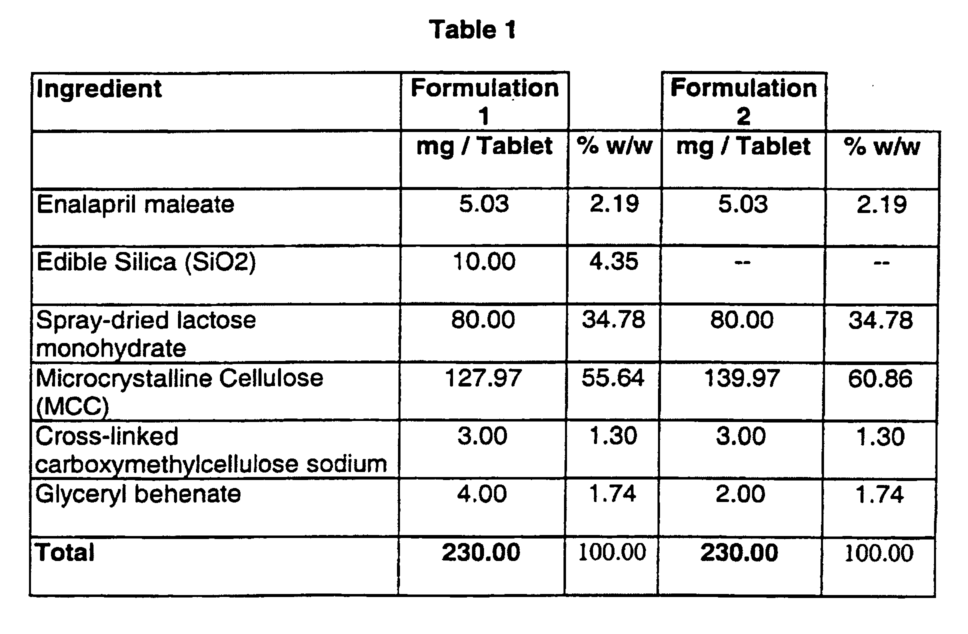

Cross-linked carboxymethyl cellulose was dried for 1 hr at 70ºC, mixed with lactose monohydrate and microcrystalline cellulose and sieved through British Standard Sieve (BSS) No. 30. Enalapril maleate and Si02 were passed through a BSS No.60 sieve, and the sieved materials were mixed together. Glyceryl behenate was passed through a BSS No. 60 sieve and blended with the mixture. The powder mixture was then compressed into tablets of average weight 230mg.

The tablets were packed in high density polyethylene bottles with child resistant closure caps and stored at 60°C for 15 days. A stability indicating assay procedure was used to determine the drug content and the total related substances (Total RS). The total RS values reduced from 6.33% (formulation 2) to 1.93% (formulation 1) when the desiccant was added to the formulation. The results indicate that the addition of an edible desiccant resulted in an improved stability of the composition. As per the pharmacopeal limits for enalapril maleate tablets, the total RS should not be more than 5%.

## Claims

1. A stable oral pharmaceutical composition in the form of tablets or capsules comprising:
(a) enalapril maleate as an active ingredient;
(b) a stabilizer consisting essentially of an edible silica dessicant in an amount ranging from 2 to 15% by weight of the total weight of the composition; and
(c) microcrystalline cellulose as an excipient.

2. The composition of claim 1 wherein the pharmaceutical composition contains a lubricant selected from the group consisting of zinc stearate and glyceryl behenate.

3. The composition of claim 1 wherein the pharmaceutical composition contains glyceryl behenate as an excipient.

## Patentansprüche

1. Stabile orale pharmazeutische Zusammensetzung in der Form von Tabletten oder Kapseln, enthaltend:
(a) Enalaprilmaleat als einen aktiven Inhaltsstoff;
(b) einen Stabilisator im Wesentlichen bestehend aus essbarem wasserentziehendem Siliziumdioxid in einer Menge im Bereich von 2 bis 15 Gew.-% des Gesamtgewichtes der Zusammensetzung; und
(c) mikrokristalline Cellulose als Träger.

2. Zusammensetzung nach Anspruch 1, wobei die pharmazeutische Zusammensetzung ein Gleitmittel ausgewählt aus der Gruppe bestehend aus Zinkstearat und Glycerylbehenat enthält.

3. Zusammensetzung nach Anspruch 1, wobei die pharmazeutische Zusammensetzung Glycerylbehenat als einen Träger enthält.

## Revendications

1. Composition pharmaceutique orale stable sous la forme de comprimés ou de gélules comprenant :
(a) un maléate d'énalapril comme ingrédient actif;
(b) un stabilisant composé essentiellement d'un dessicant de silice alimentaire dans une quantité se trouvant dans la plage de 2 à 15% en poids du poids total de la composition ; et
(c) de la cellulose microcristalline comme excipient.

2. Composition de la revendication 1 dans laquelle la composition pharmaceutique contient un lubrifiant choisi parmi le groupe composé de stéarate de zinc et de béhénate de glycéryle.

3. Composition de la revendication 1 dans laquelle la composition pharmaceutique contient du béhénate de glycéryle comme excipient.
